# EUROPÄISCHE PATENTANMELDUNG

(11) **EP 1 997 536 A2**
(43) Veröffentlichungstag der Anmeldung: **03.12.2008**
(21) Anmeldenummer: 08010713.9
(22) Anmeldetag: 06.05.2004
(51) Int. Cl.: A61Q 5/02, A61K 8/46, A61K 8/02, A61K 8/11, A61K 8/37, A61K 8/44, A61K 8/49, A61K 8/60, A61Q 13/00, A61Q 19/10, C11D 3/50, C11D 17/00

(54) **Tensidmischungen zur Verbesserung der Deposition von Aktivstoffen und Verminderung der Hautreizwirkung**

(30) Priorität: 07.05.2003 WO PCT/EP03/04788
(62) Teilanmeldung aus: 04739148.7
(71) Anmelder: KEMIRA PIGMENTS OY, 00180 Helsinki (FI)
(72) Erfinder: Dahms, Gerd, 47138 Duisburg (DE)
(74) Vertreter: Bublak, Wolfgang

(57) **Zusammenfassung**

Verwendung von Mischungen und Tensiden, die multilamellare flüssigkristalline Strukturen ausbilden, wobei die Tenside ausgewählt sind aus mindestens zwei der folgenden Gruppen, gebildet aus Isethionaten, Tauraten, Sarcosinaten, Acyllactylaten, Acylglutamaten, Fettsäure-Protein-Kondensaten, PEG-Stearaten, PEG-Distearaten mit einem HLB-Wert kleiner 10, Alkylpolyglykosiden und Betaine zur Verminderung der Hautreizwirkung von kosmetischen und/oder pharmazeutischen Formulierungen, die auf die Haut aufgebracht werden und der Verbesserung der Haarstruktur.

## Beschreibung

Die Erfindung betrifft die Verwendung von Mischungen von Tensiden zur Verminderung der Hautreizwirkung von kosmetischen und/oder pharmazeutischen Formulierungen, die auf die Haut aufgebracht werden. Die Tensidmischungen sollen zudem zur gezielten Verkapsulierung und Verbesserung der Aufbringung von Aktivstoffen auf die Haut dienen. Aktivstoffe sind dabei insbesondere organische Lichtschutzfilter, Antischuppenwirkstoffe, Keratolytika und weitere pharmazeutische Wirkstoffe, Antioxidantien, Pflanzenextrakte, Öle, Wachse und Gemische davon.

Viele kosmetische und pharmazeutische Zusammensetzungen enthalten Aktivstoffe, die in einer rinse-off-Formulierung auf die Haut aufgebracht werden. Während die rinse-off-Formulierung nach der Anwendung wieder von der Haut abgespült wird, sollen die Aktivstoffe auf der Haut deponiert werden. Üblicherweise werden die Aktivstoffe den Zusammensetzungen, beispielsweise Shampoos, Duschgelen, Gesichtsreinigern oder festen oder flüssigen Seifen, direkt beigemischt. Diese Vorgehensweise hat den Nachteil, dass meist bei der Anwendung nur geringe Mengen der Aktivstoffe auf der Haut verbleiben, die dort ihre Wirkung entfalten können. Der größte Teil der Aktivstoffe wird in der Regel mit der rinse-off-Formulierung wieder abgespült. Dies führt dazu, dass große Mengen an kostenintensiven Aktivstoffen in die Formulierungen eingebracht werden müssen, um eine gewünschte Wirkung zu erzielen. Beim Einsatz geeignet hoher Mengen der Aktivstoffe in den rinse-off-Formulierungen beobachtet man bei der Anwendung der Formulierungen jedoch häufig eine Hautreizung, die unerwünscht ist.

Es besteht daher Nachfrage nach rinse-off-Formulierungen, die es einerseits erlauben, die Einsatzmenge an Aktivstoffen gering zu halten und somit Kosten einzusparen, die zum anderen aber dennoch eine sehr gute Wirksamkeit der Aktivstoffe erlauben und eine verminderte Hautreizung bei der Anwendung bewirken.

Wenn die Wirksamkeit der Aktivstoffe beim Einsatz gesteigert werden könnte, wäre es möglich, mit geringeren Einsatzmengen auszukommen, so dass die kosmetischen oder pharmazeutischen Formulierungen kostengünstiger hergestellt werden könnten.

Aufgabe der vorliegenden Erfindung ist die Bereitstellung von kosmetischen und/oder pharmazeutischen rinse-off-Formulierungen, die die Nachteile der bekannten Formulierungen vermeiden und insbesondere eine verbesserte Aufbringung von Aktivstoffen auf die Haut erlauben. Zudem sollen sie vorzugsweise die Hautreizwirkung der Formulierungen vermindern.

Es soll möglich sein, mit geringen Mengen an Aktivstoffen in den Formulierungen eine gewünschte Wirkung in ausreichendem Maße zu erreichen.

Die Aufgaben werden erfindungsgemäß gelöst durch die Verwendung von Mischungen von Tensiden, die multilamellare flüssigkristalline Strukturen ausbilden, wobei die Tenside ausgewählt sind aus mindestens zwei der folgenden Gruppen, gebildet aus Isethionaten, Tauraten, Sarcosinaten, Acyllactylaten, Acylglutamaten, Fettsäure-Protein-Kondensaten, PEG-Stearaten, PEG-Distearaten mit einem HLB-Wert kleiner 10, Alkylpolyglykosiden und Betainen zur Verminderung der Hautzreizwirkung von kosmetischen und/oder pharmazeutischen Formulierungen, die auf die Haut aufgebracht werden, sowie zur Verbesserung der geschädigter Haarstrukturen.

Die Aufgaben werden zudem erfindungsgemäß gelöst durch Verwendung von Mischungen von Tensiden, die multilamellare flüssigkristalline Strukturen ausbilden, wobei die Tenside ausgewählt sind aus mindestens zwei der folgenden Gruppen, gebildet aus Isethionaten, Tauraten, Sarcosinaten, Acyllactylaten, Acylglutamaten, Fettsäure-Protein-Kondensaten, PEG-Stearaten, PEG-Distearaten mit einem HLB-Wert kleiner 10, Alkylpolyglykosiden und Betainen in kosmetischen und/oder pharmazeutischen Formulierungen, die Aktivstoffe enthalten, die ausgewählt sind aus organischen Lichtschutzfiltern, Antischuppenwirkstoffen, Keratolytika und weiteren pharmazeutischen Wirkstoffen, Antioxidantien, Pflanzenextrakten, Ölen, Wachsen und Mischungen davon, zur Verbesserung der Aufbringung der Aktivstoffe auf die Haut.

Es wurde erfindungsgemäß gefunden, dass eine spezifische Kombination von Tensiden zur Verminderung der Hautreizwirkung von kosmetischen und/oder pharmazeutischen Formulierungen und zur Verbesserung der Aufbringung von Aktivstoffen auf die Haut führt.

Die Aufgabe wird zudem erfindungsgemäß gelöst durch eine Mischung aus

20 bis 80 Gew.-% einer Mischung von Tensiden, die multilamellare flüssigkristalline Strukturen ausbilden, wobei die Tenside ausgewählt sind aus mindestens zwei der folgenden Gruppen, gebildet aus Isethionaten, Tauraten, Sarcosinaten, Acyllactylaten, Acylglutamaten, Fettsäure-Protein-Kondensaten, PEG-Stearaten, PEG-Distearaten mit einem HLB-Wert kleiner 10, Alkylpolyglykosiden und Betainen und 10 bis 80 Gew.-% Wasser, Polyol oder Mischungen davon.

Ferner wird die Aufgabe erfindungsgemäß gelöst durch eine kosmetische und/oder pharmazeutische, rinse-off-Formulierung, enthaltend 0,2 bis 20 Gew.-% einer wie vorstehend definierten Mischung, 0,1 bis 50 Gew.-% Aktivstoffe, die ausgewählt sind aus organischen Lichtschutzfiltern, Antischuppenwirkstoffen, Keratolytika wie Salicylsäure und weiteren pharmazeutischen Wirkstoffen, Antioxidantien, Pflanzenextrakten, Ölen, Wachsen und Gemischen davon.

Die Aufgabe wird ferner gelöst durch Verwendung einer wie vorstehend beschriebenen Mischung oder Formulierung zur Herstellung von Haar- und/oder Hautreinigungsmitteln.

Die Aufgabe wird zudem durch derartige Haar- und/oder Hautreinigungsmittel gelöst, die eine wie vorstehend definierte Mischung oder Formulierung enthalten.

Gemäß der Erfindung werden Mischungen von Tensiden eingesetzt, die multilamellare flüssigkristalline Strukturen ausbilden. Derartige Strukturen können lichtmikroskopisch mit einem Polarisationsmikroskop bestimmt werden. Ferner können multilamellare flüssigkristalline Strukturen durch TEM- oder TEM-Gefrierbruch-Technik bestimmt werden. Dem Fachmann sind entsprechende Techniken bekannt.

Die Mischungen der Tenside werden so ausgewählt, dass eine multilamellare flüssigkristalline Struktur ausgebildet wird. Die Auswahl geeigneter Mengen der Tenside ist durch einfache Handversuche möglich.

Bei der mulitlamellaren flüssigkristallinen Struktur handelt es sich beim Einsatz der Tenside in einem Lösungsmittel um lyotrope multilamellare flüssigkristalline Phasen.

Die Mischungen von Tenside enthalten mindestens zwei der folgenden Gruppen; gebildet aus Isethionaten, Tauraten, Sarcosinaten, Acyllactolaten, Acylglutamaten, Fettsäure-Protein-Kondensaten, PEG-Stearaten, PEG-Distearaten mit einem HLB-Wert kleiner 10, Alkylpolyglykosiden und Betainen. So können beispielsweise jeweils ein Vertreter der Isethionate und Taurate miteinander kombiniert werden. Es müssen in den Tensidmischungen mindestens jeweils ein Vertreter aus zwei unterschiedlichen der vorstehend genannten Gruppen vorliegen. Dabei liegen gemäß einer Ausführungsform der Erfindung in den Mischungen Tenside aus mindestens drei der vorstehend genannten Gruppen vor. Dabei liegen besonders bevorzugt Tenside aus den Gruppen der Isethionate, Acyllactylate, Alkylglucoside und Acylglutamate in den Tensidmischungen vor.

Sofern gemäß einer Ausführungsform der Erfindung Tensiden aus mindestens zwei der angegebenen Gruppen enthalten sind, enthalten die Mischung besonders bevorzugt Tenside aus mindestens zwei der folgenden Gruppen, gebildet aus Isethionaten, Acyllactylaten und Acylglutamaten oder aus den Gruppen der Isethionate, Acyllactylate und Alkylglucoside.

Aus den jeweiligen Gruppen können ein oder mehrere unterschiedliche Verbindungen in den erfindungsgemäß eingesetzten Tensidmischungen enthalten sein.

Vorzugsweise liegen in der Tensidmischung Tenside aus den mindestens zwei bzw. mindestens drei Gruppen in Mengen von jeweils mindestens 2 Gew.-% vor. Dies bedeutet, dass jede der unterschiedlichen vorgenannten Gruppen in den Tensidmischungen zu mindestens 10 Gew.-% enthalten ist, bezogen auf das Gemisch der gesamten in der Mischung eingesetzten Tenside. Dies kann sich auch auf die gesamten in einer Mischung, Formulierung oder einem Haar- und/oder Hautreinigungsmittel enthaltenden Tenside beziehen.

Von den genannten Gruppen von Tensiden sind insbesondere die Taurate, Sarcosinate, Acyllactylate und Acylglutamate besonders hautaffin und ziehen daher besonders gut auf die Haut auf.

Erfindungsgemäß bevorzugte Isethionate sind beispielsweise Ammonium, Kalium oder Natrium Cocoyl oder Lauryl Isethionate. Ein besonders bevorzugter Vertreter ist Natrium Cocoylisethionat. Bevorzugte Taurate sind Ammonium, Kalium oder Natrium Methyl Cocoate Taurate. Bevorzugte Sarcosinate sind Ammonium, Kalium oder Natrium Cocoyl oder Lauryl Sarcosinate. Bevorzugte Acyllactylate und Acylglutamate weisen einen C 8-18 Acylrest auf. Besonders bevorzugte Vertreter sind hier Lauroyl lactylate und Lauroyl glutamate. (Pationic 138A, Protelan AGL 95). Bevorzugte Fettsäure-Protein-Kondensate sind Sodium Cocoyl Hyrolyzed Wheat Protein.

Gemäß einer besonders bevorzugten Ausführungsform der Erfindung werden Natrium Lauroyl lactylate, Natrium Cocoyl Isethionate und Natrium Lauroyl glutamate (Pationic 138A, Protelan AGL 95, Tauranol I-78) als Tensidmischung eingesetzt.

Gemäß einer weiteren Ausführungsform wird Natrium Lauroyl glutamate durch Laurylglucoside ersetzt.

Gemäß einer Ausführungsform wird aus jeder der vorstehend genannten Gruppen jeweils nur maximal ein Tensid in den Tensidmischungen eingesetzt. Dabei liegen in den Tensidmischungen bevorzugt von jeder Gruppe mindestens 1 Gew.-%, besonders bevorzugt mindestens 15 Gew.-%, insbesondere mindestens 20 Gew.-% vor, bezogen auf die Mischung der Tenside.

Die Tenside der Tensidmischungen sind vorzugsweise so ausgewählt, dass in Wasser oder Polyol multilamellare Vesikel erhältlich sind, die einen mittleren Durchmesser von weniger als 15µm, besonders bevorzugt weniger als 10µm, insbesondere weniger als 5µm aufweisen. Werden die Tensidmischungen in Detergentienlösungen wie z.B. Natrium Laurylether Sulfate und/oder Betain gemischt, so verringern sich die Vesikel auf einen mittleren Durchmesser von weniger als 500 nm, besonders bevorzugt weniger als 200 nm, insbesondere weniger als 100 nm aufweisen. Es sind damit klare Produkte (im Unterschied zu beispielsweise Perlglanzprodukten) zugänglich. Die erfindungsgemäßen Tensidmischungen werden vorzugsweise in wässrigen Detergentien-Systemen eingesetzt.

Die erfindungsgemäß eingesetzten Mischungen von Tensiden werden bevorzugt in einer Mischung aus 20 bis 90 Gew.-%, vorzugsweise 40 bis 80 Gew.-%, insbesondere 50 bis 70 Gew.-% einer Mischung der Tenside, wie sie vorstehend beschrieben ist, und

10 bis 80 Gew.-%, vorzugsweise 20 bis 60 Gew.-%, insbesondere 30 bis 50 Gew.-% Wasser, Polyol oder Mischungen davon eingesetzt. Diese Mischung besteht vorzugsweise aus den genannten Mischungen von Tensiden, Wasser und/oder Polyol.

Erfindungsgemäß werden dabei solche Tenside eingesetzt, die zur Ausbildung von lyotropen lamellaren liquidkristallinen Phasen fähig sind. Die Bildung von liquidkristallinen Strukturen hängt im Wesentlichen von der Geometrie der Tenside ab.

Dabei spielt das Verhältnis von hydrophilem zu hydrophobem Rest eine wichtige Rolle. Tenside mit einer raumerfüllenden hydrophilen Gruppe und kleinem hydrophoben Rest bilden häufig Mizellen aus. Mizellen liegen jedoch in einem dynamischen Gleichgewicht vor und bauen sich ständig ab und wieder auf. Daher sind Mizellen weniger als Speicher für andere Inhaltsstoffe geeignet. Mit kleiner werdendem hydrophilen Rest bilden die Tenside stäbchenförmige Mizellen, Vesikel-Doppelschichten und Sandwich-Doppelschichten aus. Erfindungsgemäß werden nun Tenside eingesetzt, die in einer lyotropen lamellaren liquidkristallinen Phase vorliegen können. Im lyotropen Zustand werden z.B. zwischen den hydrophoben Resten bzw. Köpfen der Tenside Aktivstoffe gespeichert. Der hydrophile Teil des Tensids kann je nach gewünschter Haftung zu einem späteren Substrat variiert werden. Beispielsweise kann der hydrophobe Teil variiert werden zur Haftung an der menschlichen Haut oder an Textilfasern.

Es können auch weitere Tenside mitverwendet werden.

Geeignete weitere Tenside, die lyotrope lamellare liquidkristalline Phasen ausbilden, sind dem Fachmann bekannt. Es können natürliche oder synthetische Produkte eingesetzt werden. Auch der Einsatz von Tensidgemischen ist möglich. Beispiele geeigneter Tenside sind die physiologischen Gallensalze wie Natriumcholat, Natriumdehydrocholat, Natriumdeoxucholat, Natriumglykocholat, Natriumtaurocholat. Tierische und pflanzliche Phospholipide wie Lecithine mit ihren hydrierten Formen sowie Polypeptide wie Gelatine mit ihren modifizierten Formen können ebenso verwendet werden.

Als synthetische grenzflächenaktive Substanzen eignen sich die Salze der Sulfobernsteinsäureester, Polyoxyethylensorbitanester, Sorbitanester, Polyoxyethylenfettalkoholether, Polyoxyethylenfettsäureester sowie .entsprechende Mischkondensate von Polyoxyethylen- mit Polyoxypropylenethern, ethoxylierte gesättigte Glyceride, partielle Fettsäure-Glyceride und Polyglyceride.

Beispiele geeigneter Tenside sind ferner Glycerinester, Polyglycerinester, Sorbitanester, Sorbitolester, Fettalkohole, Propylenglykolester, Alkylglucosidester, Zuckerester, Lecithin, Silikoncopolymere, Wollwachs und deren Mischungen oder Derivate davon. Glycerinester, Polyglycerinester, Alkoxylate und Fettalkohole sowie Isoalkohole können sich beispielsweise ableiten von Rhizinusfettsäure, 12-Hydroxystearinsäure, Isostearinsäure, Ölsäure, Linolsäure, Linolensäure, Stearinsäure, Myristinsäure, Laurinsäure und Caprinsäure. Neben den genannten Estern können auch Succinate, Amide oder Ethanolamide der Fettsäuren vorliegen. Als Fettsäurealkoxylate kommen insbesondere die Ethoxylate, Propoxylate oder gemischten Ethoxylate/Propoxylate in Betracht.

Für auf die menschliche Haut aufzubringende Zusammensetzungen kommen insbesondere Lactylate, Glutamate, Ethoxylate von Alkoholen oder Glykolen, Betaine, amphiphile Coemulgatoren wie Sorbitanmonostearat und Fettalkohole, Fettsäurekondensate, Sarkosinate, Proteinfettsäurekondensate, Sulfosuccinate und Ethercarboxylate zum Einsatz.

Die lyotrope lamellare liquidkristalline Phase wird dabei vorzugsweise unter Einsatz von Wasser, Alkoholen, Polyolen oder Gemischen davon ausgebildet. Hierbei orientieren sich die hydrophilen Anteile der Tenside zur Wasser-, Alkohol-, Polyol-Phase oder Mischphase davon aus, während die hydrophoben Anteile in der lamellaren Struktur zueinander weisen.

Die Mischung aus Tensidmischung und Wasser/Polyol, die in lyotroper multilamellarer flüssigkristalliner Struktur vorliegt, kann in beliebige kosmetische und/oder pharmazeutische rinse-off-Formulierungen eingebracht werden. Sie dient dabei insbesondere zur Verminderung der Hautreizwirkung der kosmetischen' und/oder pharmazeutischen Formulierungen, oder darüber hinaus, falls Aktivstoffe in den Formulierungen vorliegen, zur Verbesserung der Aufbringung der Aktivstoffe auf die Haut bei der Anwendung.

Eine derartige erfindungsgemäße kosmetische und/oder pharmazeutische flüssigkristalline rinse-off-Formulierung enthält 0,2 bis 20 Gew.-%, vorzugsweise 0,5 bis 10 Gew.-%, insbesondere 1 bis 5 Gew.-% der vorstehend beschriebenen Basismischung aus Tensidmischung und Wasser/Polyol, sowie 0,1 bis 50 Gew.-%, vorzugsweise 1 bis 20 Gew.-%, insbesondere 1,5 bis 15 Gew.-% Aktivstoffe, die ausgewählt sind aus organischen Lichtschutzfiltern, Antischuppenwirkstoffen, Keratolytica und weiteren pharmazeutischen Wirkstoffen, Antioxidantien, Pflanzenextrakten, Ölen, Wachsen und Gemischen davon. Die kosmetische und/oder pharmazeutische rinse-off-Formulierung enthält üblicherweise weitere für diese Formulierungen typische Inhaltsstoffe. Die Kombination aus Tensidmischung, Wasser/Polyol und Aktivstoffen kann aber auch, wie auch die Mischung aus Tensidmischung und Wasser/Polyol an sich, zur Herstellung von Haar- und/oder Hautreinigungsmitteln eingesetzt werden. Derartige Haar- und/oder Hautreinigungsmittel können in jeder beliebigen geeigneten Form vorliegen, beispielsweise als Shampoos, Duschgele, Gesichtsreiniger oder Seifen.

Die Erfindung betrifft auch entsprechende Haar- und/oder Hautreinigungsmittel. Diese Mittel erlauben ein Aufziehen der Aktivstoffe auf die Haut. Zudem ermöglichen sie eine Lösung der Aktivstoffe in den Mitteln und die Herabsetzung von Hautirritationen bei der Anwendung. Ferner kann häufig ein Schäumen von ansonsten nicht schäumenden Detergenzien durch Einsatz der erfindungsgemäßen Mittel erreicht werden.

Als Aktivstoffe kommen beispielsweise Öle oder Wachse in Betracht. Geeignete Öle sind beispielsweise Silikonöle und Silikonamine, bei denen es sich um aminofunktionelle Silikonöle handelt. Ferner können Esteröle, Silikonwachse, natürliche Wachse und Polyole eingesetzt werden.

Weiterhin geeignet sind kosmetische Wirkstoffe, die insbesondere oxidations- oder hydrolyseempfindlich sind wie beispielsweise Polyphenole. Hier seien genannt Catechine (wie Epicatechin, Epicatechin-3-gallat, Epigallocatechin, Epigallocatechin-3-gallat), Flavonoide (wie Luteolin, Apigenin, Rutin, Quercitin, Fisetin, Kaempherol, Rhametin), Isoflavone (wie Genistein, Daidzein, Glycitein, Prunetin), Cumarine (wie Daphnetin, Umbelliferon), Emodin, Resveratrol, Oregonin.

Geeignet sind Vitamine wie Retinol, Tocopherol, Ascorbinsäure, Riboflavin, Pyridoxin. Geeignet sind ferner Gesamtextrakte aus Pflanzen, die u.a. obige Moleküle oder Molekülklassen enthalten.

Bei den Wirkstoffen handelt es sich gemäß einer Ausführungsform der Erfindung um Lichtschutzfilter. Diese können als organische Lichtschutzfilter bei Raumtemperatur (25°C) in flüssiger oder fester Form vorliegen. Geeignete Lichtschutzfilter (UV-Filter) sind beispielsweise Verbindungen auf Basis von Benzophenon, Diphenylcyanacrylat oder p-Aminobenzoesäure. Konkrete Beispiele sind (INCI- oder CTFA-Bezeichnungen) Benzophenone-3, Benzophenone-4, Benzophenone-2, Benzophenone-6, Benzophenone-9, Benzophenone-1, Benzophenone-11, Etocrylene, Octocrylene, PEG-25 PABA, Phenylbenzimidazole Sulfonic Acid, Ethylhexyl Methoxycinnamate, Ethylhexyl Dimethyl PABA, 4-Methylbenzylidene Camphor, Butyl Methoxydibenzoylmethane, Ethylhexyl. Salicylate, Homosalate sowie Methylene-Bis-Benzotriazolyl Tetramethylbutylphenol (2,2'-Methylen-bis- {6-(2H-benzoetriazol-2-yl)-4-(1,1,3,3-tetramethylbutyl)-phenol}, 2-Hydroxy-4-methoxybenzophenon-5-sulfonsäure und 2,4,6-Trianilino-p-(carbo-2'-ethylhexyl-1'-oxi)-1,3,5-triazin.

Weitere organische Lichtschutzfilter sind Ethylhexyltriazone, Avobenzone, Ethylhexylmethoxycinnamate, Ethylhexylsalicylate, Benzotriazole und Triazine.

Gemäß einer weiteren Ausführungsform der Erfindung werden als Wirkstoffe Antischuppen-Wirkstoffe eingesetzt, wie sie üblicherweise in kosmetischen oder pharmazeutischen Formulierungen vorliegen. Ein Beispiel hierfür ist Piroctone Olamine (Octopyrox®) (1-Hydroxy-4-methyl-6-(2,4,4-dimethylpentyl)-2(1H)-pyridone); vorzugsweise in Kombination mit 2-Aminoethanol (1:1)). Weitere geeignete Mittel zur Behandlung von Hautschuppen sind dem Fachmann bekannt, z.B. Zinkpyrithion und Salicylsäure.

Als Aktivstoffe können auch beispielsweise Acylascorbate, Panthenol, Panthenylether, Ceramide/Sphingolipide oder Polyphenole eingesetzt werden.

Auch Pflanzenextrakte und Duftstoffe und Aromen können eingesetzt werden.

Unter Duftstoffen und Aromen werden erfindungsgemäß sowohl Duftöle (Fragrance) wie auch Aromastoffe (Flavour) verstanden. Es handelt sich hierbei um Riechstoffe, speziell Duftstoffe. Grundstoffe der Duftstoffe sind in der Regel etherische Öle, Blütenöle, Extrakte aus pflanzlichen und tierischen Drogen, aus Naturprodukten isolierte, chemisch veränderte (halbsynthetische) sowie rein synthetisch gewonnene Riechstoffe. Zu den Duftstoffen werden erfindungsgemäß auch Geschmacksstoffe gezählt.

Die Duftstoffe und Aromen können dabei aus einer Vielzahl pflanzlicher Ausgangsmaterialien stammen. Beispielsweise können genannt werden: Blüten, beispielsweise von Lavendel, Rosen, Jasmin, Neroli; Stängel und Blätter, beispielsweise von Geranium, Patchouli, Petitgrain, Früchte wie Anis, Koriander, Kümmel, Wacholder; Fruchtschalen, beispielsweise von Agrumen wie Bergamotte, Zitronen, Orangen; Samen wie Macis, Angelika, Sellerie, Kardamom; Wurzeln wie Angelika, Costus, Iris, Calmus; Holz wie Sandel-, Guajak-, Zedern-, Rosenholz; Kräuter und Gräser wie Estragon, Limonengras, Salbei, Thymian; Nadeln und Zweige, beispielsweise von Fichten, Tannen, Kiefern, Latschen; Harze und Balsame, beispielsweise aus Galvanum, Elemi, Benzoe, Myrrhe, Olibanum, Opoponax.

Tierische Rohstoffe sind beispielsweise Ambra, Moschus, Zibet, Castoreum.

Beispiele halb synthetischer Riechstoffe sind Isoeugenol, Vanillin, Hydroxycitronellal, Citronellol, Geranylacetat, Jonone und Methyljonone. Die vollsynthetischen Riechstoffe oder Duftstoffe sind sehr vielfältig und orientieren sich häufig an natürlichen Stoffen. Für eine Beschreibung der Duftstoffe kann beispielsweise auf Römpp, Chemielexikon, 9. Auflage, Stichworte "Parfums", "Riechstoffe", "Duftstoffe", verwiesen werden. Weitere geeignete Duftstoffe und Aromen sind dem Fachmann bekannt

Die Aktivstoffe können beispielsweise in die Räume zwischen den hydrophoben Resten der Tenside eingebracht und dort gespeichert werden. Hierdurch werden die Aktivstoffe gelöst, und ein Auskristallisieren der Aktivstoffe wird verhindert. Dies erlaubt u.a. die Herstellung von kosmetischen und/oder pharmazeutischen Formulierungen bei einem hautfreundlichen pH-Wert, und durch die Verhinderung des Auskristallisierens der Aktivstoffe wird die Hautfreundlichkeit der Zusammensetzung weiter erhöht. Die erfindungsgemäß eingesetzten Tensidmischungen mit den darin gelösten Aktivstoffen spreiten beim Aufbringen auf die Haut, so dass ein verbesserter Auftrag der Aktivstoffe auf die Haut erfolgt.

Über die Speicherwirkung hinaus erlaubt die erfindungsgemäße Vorgehensweise einen weitgehend Schutz der Aktivstoffe vor dem oxidativen Zerfall. Gegebenenfalls können noch weitere Antioxidantien zugesetzt werden.

Auch ohne den Zusatz von Antioxidantien ist der Aktivstoff in den erfindungsgemäßen Zusammensetzungen wesentlich besser gegenüber der Oxidation geschützt als in herkömmlichen Darreichungsformen..

Bezogen auf die letztendlich erhaltene Anwendungsformulierung, beispielsweise ein Shampoo, Duschgel, Duschöl, Duschbad, Badezusatz oder einen Gesichtsreiniger, liegen Lichtschutzfilter, Antischuppenwirkstoffe, Antioxidantien oder Pflanzenextrakte, vorzugsweise in einer Menge von 0,05 bis 10 Gew.-%, besonders bevorzugt 0,1 bis 5 Gew.-%, insbesondere 0,2 bis 3 Gew.-% vor.

Die erfindungsgemäßen Zusammensetzungen können neben den erfindungsgemäßen Tensidmischungen zusätzliche Emulgatoren oder Tenside enthalten, deren Hautreizwirkung durch die erfindungsgemäßen Tensidmischungen vermindert wird.

Beispiele geeigneter Substanzen sind Tenside, insbesondere Acyllactylate wie Stearoyllactylat, Isethinonate, Alkylsulfate wie Cetylsulfat, Diamideethersulfate, Alkylpolyglycoside, Phosphorsäureester, Taurate, Sulfosuccinate, Alkylsarcosinate wie Natriumlaurylsarcosinat und Alkylglutamate wie Natriumlaurylglutamat, ethoxylierte Sorbitanfettsäureester, Blockpolymere und Blockcopolymere (wie zum Beispiel Poloxamere und Poloxamine), Polyglycerinether und -ester, Lecithine verschiedenen Ursprungs (zum Beispiel Ei- oder Sojalecithin), chemisch modifizierte Lecithine (zum Beispiel hydriertes Lecithin) als auch Phospholipide und Sphingolipide, Mischungen von Lecithinen mit Phospholipiden, Sterine (zum Beispiel Cholesterin und Cholesterinderivate sowie Stigmasterin), Ester und Ether von Zuckern oder Zuckeralkoholen mit Fettsäuren oder Fettalkoholen (zum Beispiel Saccharosemonostearat), ethoxylierte Sorbitanfettsäureester, ethoxylierte Mono- und Diglyceride, ethoxylierte Lipide und Lipoide, ethoxylierte Fettalkohole oder Fettsäuren und Ladungsstabilisatoren bzw. Ladungsträger wie zum Beispiel Dicetylphosphat, Phosphatidylglycerin sowie gesättigte und ungesättigte Fettsäuren, Natriumcholat, Natriumglykolcholat, Natriumtaurocholat oder deren Mischungen, Aminosäuren oder Peptisatoren wie Natriumcitrat (siehe J. S. Lucks, B. W. Müller, R. H. Müller, Int. J. Pharmaceutics 63, Seiten 183 bis 18 (1990)), viskositätserhöhende Stoffe wie Celluloseether und -ester (zum Beispiel Methylcellulose, Hydroxyethylcellulose, Hydroxypropylcellulose, Natriumcarboxymethylcellulose), Polyvinylderivate wie Polyvinylalkohol, Polyvinylpyrrolidon, Polyvinylacetat, Alginate, Polyacrylate (zum Beispiel Carbopol), Xanthane und Pektine.

Als wässrige Phase können Wasser, wässrige Lösungen oder Mischungen von Wasser mit wassermischbaren Flüssigkeiten wie Glycerin oder Polyethylenglycol eingesetzt werden. Weitere zusätzliche Komponenten für die wässrige Phase sind beispielsweise Mannose, Glucose, Fructose, Xylose, Trehalose, Mannit, Sorbit, Xylit oder andere Polyole wie Polyethylenglykol sowie Elektrolyte wie Natriumchlorid. Diese zusätzlichen Komponenten können in einer Menge von 1 bis 30 Gew.-%, bezogen auf die wässrige Phase, eingesetzt werden.

Falls gewünscht, können ferner viskositätserhöhende Stoffe oder Ladungsträger eingesetzt werden, wie Sie in EP-B-0 605 497 beschrieben sind. Als Verdicker können zum Beispiel Polysaccharide, Polyalkylacrylate, Polyalkylcyanoacrylate, Polyalkylvinylpyrrolidone, Acrylpolymere, Polymilchsäuren oder Polylactide eingesetzt werden.

In den Zusammensetzungen können die Wasserresistenz und das Spreit- und Haftvermögen durch Silikonzusatz erhöht werden. Geeignete Silikonderivate sind dabei Dimethicon, alkyl- und arylsubstituierte Silikone, aminosubstituierte Silikonöle, Silikon-Copolyole mit Alkylpolyglykosiden, modifizierte Silikonöle usw. Alternativ oder zusätzlich können auch fluorierte Kohlenwasserstoffe eingesetzt werden.

Die erfindungsgemäßen Zusammensetzungen, insbesondere Mischungen aus den Tensidmischungen und Wasser/Polyol und die Formulierungen, die zusätzliche Aktivstoffe enthalten, werden erfindungsgemäß hergestellt, in dem man das Tensidgemisch mit dem in flüssiger Form vorliegenden Wasser oder Polyol unter Ausbildung einer lyotropen lamellaren flüssigkristallinen Phase vermischt und gegebenenfalls sodann die Aktivstoffe einmischt.

Die erfindungsgemäßen Zusammensetzungen können in Form von Emulsionen oder multiplen Emulsionen vorliegen.

Bei den Emulsionen kann es sich um O/W-Emulsionen, W/O-Emulsionen, P/O-Emulsionen oder multiple Emulsionen handeln. Die einzelnen Phasen der Emulsionen können noch übliche für die einzelnen Phasen bekannte Inhaltsstoffe aufweisen. Es kann beispielsweise auf DE-A 43 41 113, EP-B 0 605 497, EP-B-0 167 825 und US 5,885,486 verwiesen werden.

Typische weitere Inhaltsstoffe der erfindungsgemäßen Haar- und/oder Hautreinigungsmittel sind beispielsweise Betaine, APGs, anorganische und organische Verdicker, Polymere, Duftstoffe und Farbstoffe. Zudem können weitere übliche Tenside, wie anionische Tenside, kationische Tenside, amphotere Tenside und nicht-ionische Tenside eingesetzt werden, sofern sie die Wirksamkeit der erfindungsgemäß eingesetzten Tensidmischungen nicht beeinträchtigen.

Die Erfindung wird durch die nachstehenden Beispiele näher erläutert.

### Beispiele

### Beispiel 1

### Basisformulierung

30 Gewichtsteile Sodium Lauroyl Lactylate, 15 Gewichtsteile Sodium Cocoylisethionate, 30 Gewichtsteile Sodium Lauroyl Glutamate und 25 Gewichtsteile Wasser wurden zusammen auf 60 °C erwärmt und solange gerührt, bis sich ein homogenes Gemisch ergab.

Das Gemisch wies einen pH-Wert von 6,6 auf. Es wurde als Phase A in den weiteren Beispielen verwendet.

### Beispiel 2

5 % der Phase A wurden in 12 %ige wässrige Ethersulfatlösung gegeben. Diese Mischung aus 5 % der Phase A und 12 % Ethersulfat in Wasser wurde in einem human patch Test bei 20 Probanden auf die Haut aufgetragen. Zum Vergleich wurde auf andere Hautteile eine 12 %ige wässrige Ethersulfatlösung aufgetragen. Im Unterschied zur Vergleichslösung wies die Lösung, die die Phase A enthielt, ein angenehm seidiges Hautgefühl auf, und Hautirritationen konnten auf Null gesenkt werden. Im Vergleich dazu traten beim Einsatz der 12 %igen wässrigen Ethersulfatlösung allein bei den Probanden Hautirritationen auf.

Hiermit wird deutlich, dass die erfindungsgemäße Phase A das Irritationspotential von üblichen rinse-off-Produkten vermindert. Typischerweise kann die erfindungsgemäße Phase A in Mengen von etwa 1 bis 10 Gew.-% einem Shampoo oder Hautreinigungsprodukt zugesetzt werden.

### Beispiel 3

### Antischuppenshampoo

3,5 Gewichtsteile Natriumlaurylethersulfat, 3,0 Gewichtsteile der Phase A aus Beispiel 1, 0,2 Gewichtsteile Octopyrox, 3,0 Gewichtsteile NaCl und 87,3 Gewichtsteile demineralisiertes Wasser wurden für eine Minute im Turrax mit 8000 U/min bei Raumtemperatur homogenisiert, nachdem zuvor die Phase A und Octopyrox bei 60 °C vermischt wurden. Es ergab sich ein pH-Wert von 7,2, der sodann auf 6,6 eingestellt wurde. Mit der erfindungsgemäßen Formulierung war es möglich, einen leicht sauren pH-Wert einzustellen, ohne dass Octopyrox auskristallisierte.

Eine Vergleichszusammensetzung wurde aus 3,5 Gewichtsteilen Natriumlaurylethersulfat, 0,5 Gewichtsteilen Octopyrox, 3 Gewichtsteilen NaCl und 93 Gewichtsteilen demineralisiertem Wasser hergestellt. Der pH-Wert betrug 9,0 und konnte nicht neutral eingestellt werden, da sonst Octopyrox auskristallisiert wäre. Durch den basischen pH-Wert ist die Zusammensetzung nicht hautfreundlich.

Das erfindungsgemäße Antischuppenshampoo und das Vergleichsshampoo wurden zur Verminderung der Schuppendichte bei 30 Probanden in einem Test eingesetzt, wobei die Probanden zu Beginn der Behandlung, nach 14 und nach 28 Tagen untersucht wurden. Dabei wurde zum einen als objektiver Test die Schuppenmenge bestimmt, zum andern wurden die Probanden als subjektiver Test nach Jucken, Hautrötung und weiteren Effekten befragt.

Es zeigte sich, dass das erfindungsgemäße Produkt im Anwendungstest sowohl in Bezug auf die verbleibende Schuppenmenge als auch in Bezug auf die verminderte Hautreizung dem Vergleichsshampoo deutlich überlegen war.

### Beispiel 4

### Basisformulierung

22 Gewichtsteile Sodium Lauroyl Lactylate, 8 Gewichtsteile Sodium Cocoyl Iesthionate und 15 Gewichtsteile Lauryl Glucoside wurden mit 55 Gewichtsteilen Wasser zusammen auf 60 °C erhitzt und unter Rühren homogenisiert.

Die folgenden Seiten 15 bis 17 betreffen bevorzugte Ausführungsformen der Erfindung.
1. Verwendung von Mischungen von Tensiden, die multilamellare flüssigkristalline Strukturen ausbilden, wobei die Tenside ausgewählt sind aus mindestens zwei der folgenden Gruppen, gebildet aus Isethionaten, Tauraten, Sarcosinaten, Acyllactylaten, Acylglutamaten, Fettsäure-Prötein-Kondensaten, PEG-Stearaten, PEG-Distearaten mit einem HLB-Wert kleiner 10, Alkylpolyglykosiden und Betaine zur Verminderung der Hautzreizwirkung von kosmetischen und/oder pharmazeutischen Formulierungen, die auf die Haut aufgebracht werden und der Verbesserung der Haarstruktur.
2. Verwendung von Mischungen von Tensiden, die multilamellare flüssigkristalline Strukturen ausbilden, wobei die Tenside ausgewählt sind aus mindestens zwei der folgenden Gruppen, gebildet aus Isethionaten, Tauraten, Sarcosinaten, Acyllactylaten, Acylglutamaten, Fettsäure-Protein-Kondensaten, PEG-Stearaten, PEG-Distearaten mit einem HLB-Wert kleiner 10, Alkylpolyglykosiden und Betainen in kosmetischen und/oder pharmazeutischen Formulierungen, die Aktivstoffe enthalten, die ausgewählt sind aus organischen Lichtschutzfiltern, Antischuppenwirkstoffen und weiteren pharmazeutischen Wirkstoffen, Keratolytika, Antioxidantien, Pflanzenextrakten, Ölen, Wachsen und Mischungen davon, zur Verbesserung der Aufbringung der Aktivstoffe auf die Haut und das Haar.
3. Verwendung nach Anspruch 1 oder 2, **dadurch gekennzeichnet, dass** die Mischungen Tenside aus mindestens drei der Gruppen enthalten.
4. Verwendung nach Anspruch 1 oder 2, **dadurch gekennzeichnet, dass** die Mischungen Tenside aus mindestens zwei der folgenden Gruppen, gebildet aus Isethionaten, Acyllactylaten, Alkylpolyglykosiden und Acylglutamaten enthalten.
5. Verwendung nach Anspruch 3, **dadurch gekennzeichnet, dass** die Mischungen Tenside aus den Gruppen der Isethionate, Acyllactylate und Acylglutamate oder aus den Gruppen der Isethionate, Acyllactylate und Alkylglucoside enthalten.
6. Verwendung nach einem der Ansprüche 1 bis 5, **dadurch gekennzeichnet, dass** in der Tensidmischung Tenside aus mindestens zwei bzw. mindestens drei Gruppen in Mengen von jeweils mindestens 10 Gew.-% vorliegen.
7. Verwendung nach einem der Ansprüche 1 bis 6, **dadurch gekennzeichnet, dass** die kosmetischen und/oder pharmazeutischen Formulierungen rinse-off-Formulierungen sind.
8. Mischung aus 20 bis 90 Gew.-% einer Mischung von Tensiden, die multilamellare flüssigkristalline Strukturen ausbilden, wobei die Tenside ausgewählt sind aus mindestens zwei der folgenden Gruppen, gebildet aus Isethionaten, Tauraten, Sarcosinaten, Acyllactylaten, Acylglutamaten, Fettsäure-Protein-Kondensaten, PEG-Stearaten, PEG-Distearaten mit einem HLB-Wert kleiner 10, Alkylpolyglykosiden und Betainen 10 bis 80 Gew.-% Wasser, Polyol oder Mischungen davon.
9. Mischung nach Anspruch 8, **dadurch gekennzeichnet, dass** in der Mischung von Tensiden Tenside aus mindestens zwei der folgenden Gruppen, gebildet aus Isethionaten, Acyllactylaten, Alkylpolyglykosiden und Acylglutamaten enthalten.
10. Mischung nach Anspruch 8, **dadurch gekennzeichnet, dass** die Mischung von Tensiden Tenside aus den Gruppen der Isoethionate, Acyllactylate und Acylglutamate oder aus den Gruppen der Isethionate, Acyllactylate und Alkylglukoside enthält.
11. Kosmetische und/oder pharmazeutische, rinse-off-Formulierung, enthaltend 0,2 bis 20 Gew.-% einer Mischung gemäß einem der Ansprüche 8 bis 10 und 0,1 bis 50 Gew.-% Aktivstoffe, die ausgewählt sind aus organischen Lichtschutzfiltern, Antischuppenwirkstoffen und weiteren pharmazeutischen Wirkstoffen, Antioxidantien, Pflanzenextrakten, Ölen, Wachsen und Gemischen davon.
12. Verwendung einer Mischung nach einem der Ansprüche 8 bis 10 oder einer Formulierung nach Anspruch 11 zur Herstellung von Haar- und/oder Hautreinigungsmitteln.
13. Haar- und/oder Hautreinigungsmittel, enthaltend eine Mischung nach einem der Ansprüche 8 bis 10 oder eine Formulierung nach Anspruch 11.

## Patentansprüche

1. Verwendung von Tensidmischungen aus Isethionaten, Acyllactylaten und Acylglutamaten oder aus Isethionaten, Acyllactylaten und Alkylglucosiden, die multilamellare flüssigkristalline Strukturen ausbilden, zur Verbesserung der Haarstruktur.

2. Verwendung nach Anspruch 1, **dadurch gekennzeichnet, dass** in der Tensidmischung Tenside aus den mindestens drei Gruppen in Mengen von jeweils mindestens 10 Gew.-% vorliegen.

3. Verwendung einer Mischung aus 20 bis 90 Gew.-% einer Tensidmischung aus Isethionaten, Acyllactylaten und Acylglutamaten oder Isethionaten, Acyllactylaten und Alkylglucosiden, die multilamellare flüssigkristalline Strukturen ausbilden, und 10 bis 80 Gew.-% Wasser, Polyol oder Mischungen davon, oder einer kosmetischen und/oder pharmazeutischen rinse-off-Formulierung, enthaltend 0,2 bis 20 Gew.-% besagter Mischung und 0,1 bis 50 Gew.-% Aktivstoffe, die ausgewählt sind aus organischen Lichtschutzfiltern, Antischuppenwirkstoffen und weiteren pharmazeutischen Wirkstoffen, Antioxidantien, Pflanzenextrakten, Ölen, Wachsen und Gemischen davon, zur Herstellung von Haarreinigungsmitteln.

4. Verwendung nach Anspruch 3, **dadurch gekennzeichnet, dass** in der Tensidmischung Tenside aus den mindestens drei Gruppen in Mengen von jeweils mindestens 10 Gew.-% vorliegen.
